(19) Europäisches Patentamt
European Patent Office
Office européen des brevets

(11) EP 0 647 218 B1

(12) **FASCICULE DE BREVET EUROPEEN**

(45) Date de publication et mention de la délivrance du brevet: **27.05.1998 Bulletin 1998/22**

(21) Numéro de dépôt: **93913143.9**

(22) Date de dépôt: **16.06.1993**

(51) Int Cl.6: **C07C 319/20**, C07C 321/18, C07C 321/20, C08F 28/04, C07D 333/08

(86) Numéro de dépôt international: **PCT/FR93/00588**

(87) Numéro de publication internationale: **WO 94/00426 (06.01.1994 Gazette 1994/02)**

(54) **PROCEDE DE PREPARATION DE COMPOSES ORGANIQUES OU DE POLYMERES CONTENANT DU SOUFRE PAR METATHESE**

VERFAHREN ZUR HERSTELLUNG VON SCHWEFEL ENTHALTENDEN ORGANISCHEN VERBINDUNGEN ODER POLYMEREN DURCH METATHESE

METHOD FOR METATHETICALLY PREPARING SULPHUR-CONTAINING POLYMERS OR ORGANIC COMPOUNDS

(84) Etats contractants désignés: **AT BE CH DE DK ES FR GB GR IE IT LI LU MC NL PT SE**

(30) Priorité: **26.06.1992 FR 9207898**

(43) Date de publication de la demande: **12.04.1995 Bulletin 1995/15**

(73) Titulaire: **ELF AQUITAINE**
**92400 Courbevoie (FR)**

(72) Inventeurs:
- **BASSET, Jean-Marie**
  **F-69100 Villeurbanne (FR)**
- **COUTURIER, Jean-Luc**
  **F-74520 Valleiry (FR)**
- **LECONTE, Michel**
  **F-69100 Villeurbanne (FR)**
- **OLLIVIER, Jean**
  **F-64260 Arudy (FR)**
- **TANAKA, Katsumi**
  **Chofu-shi, Tokyo 182 (JP)**

(74) Mandataire: **Clisci, Serge et al**
**S.A. FEDIT-LORIOT & AUTRES**
**CONSEILS EN PROPRIETE INDUSTRIELLE**
**38, Avenue Hoche**
**75008 Paris (FR)**

(56) Documents cités:
**EP-A- 0 191 675** **EP-A- 0 283 400**

- **CHEMICAL ABSTRACTS, vol. 115, no. 23, 9 Décembre 1991, Columbus, Ohio, US; abstract no. 255485x, A.V. ANISIMOV ET AL. 'On the possibility of metathesis of diallylsulfide and diallylsulfone on heterogeneous alumina-rhenium catalyst.' page 796 ;colonne 2 ;**
- **ANGEWANDTE CHEMIE. INTERNATIONAL EDITION vol. 31, no. 5, Mai 1992, WEINHEIM DE pages 628 - 631 J.-L. COUTURIER ET AL. 'A Cyclometallated Aryloxy(chloro)neopentylidenetungsten Complex: A Highly Active and Stereoselective Catalyst for the Metathesis of cis- and trans-2-Pentene, Norbonene, 1-Methylnorbonene, and Ethyl Oleate'**
- **JOURNAL OF THE CHEMICAL SOCIETY, CHEMICAL COMMUNICATIONS no. 24, 15 Décembre 1985, LETCHWORTH GB pages 1816 - 1817 F. QUIGNARD ET AL. 'Synthesis and Catalytic Properties of W(OAr)2Cl2(CHCMe3)(OR2) and W(OAr)2Cl(CHCMe3)(CH2CMe3)(OR2) (Ar = 2,6-disubstituted phenyl; R = Et or Pri), New Uni-component Catalysts for Metathesis of Acyclic and Cyclic Olefins, with or without Functional Groups'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 647 218 B1

Printed by Jouve, 75001 PARIS (FR)

## Description

L'invention se rapporte à un procédé de préparation de composés organiques mono- ou polymères, porteurs d'une ou de plusieurs doubles liaisons dans une chaîne hydrocarbonée contenant un ou plusieurs atomes de soufre. De tels composés, grâce à leurs insaturations et à la présence de la fonction soufrée peuvent être utilisés comme intermédiaires en synthèse organique et comme co-monomères, agents de liaison ou de réticulation, ou modificateurs dans des réactions de polymérisation. Les polymères contenant du soufre sont particulièrement intéressants par leurs résistances aux solvants, à la chaleur et à l'inflammation. L'invention comprend également les nouveaux produits en question.

La préparation de composés de ce genre s'avère difficile par les moyens classiques de la synthèse organique. Jusqu'à présent, dans la littérature technique, rien n'indique que la réaction de métathèse pourrait s'appliquer à des oléfines acycliques ou cycliques, comportant l'élément soufre dans leur chaîne hydrocarbonée ; on peut se référer par exemple à l'ouvrage général et très complet de K. J. Ivin : "Olefin Metathesis", Academic Press, London (1983), à l'article de J. Otton paru dans "Informations Chimie", n° 201 (Mai 1980) pages 161 à 168, ou à l'article de J.C. Mol portant sur la métathèse des oléfines comportant des groupes fonctionnels et paru dans l'ouvrage : "Olefin Metathesis and Polymerization Catalysts", (Editeurs : Y. Imamoglu, B. Zumreoglu-Karan et A. J. Amass), Kluwer Academic Publishers, Dordrecht (1990), pages 115 à 140).

On connaît par ailleurs de FR-A-2 577 216 (qui correspond à EP-A-0 191 675), de FR-A-2 612 422 (qui correspond à EP-A-0 283 400) et de l'article de J.-L. Couturier et al., Angew. Chem. Int. Ed. Engl. 31, pages 628-631 (mai 1992) l'utilisation des catalyseurs à base de tungstène de formules (15), (16) et (17) ci-après pour effectuer la métathèse d'oléfines cycliques ou acycliques. Cependant cet art antérieur, qui indique que les oléfines de départ peuvent comporter des groupements fonctionnels : carboxyles, hydroxyles, amines, amides, nitriles, silyles, halogènes, éthers ou esters, ne décrit ni ne suggère que lesdites oléfines peuvent être soufrées.

De plus, quelques travaux de l'art antérieur ont montré que l'addition de composés organiques soufrés, en particulier de sulfures organiques, à des systèmes catalytiques de métathèse, pouvait ralentir ; et même inhiber totalement, la réaction de métathèse d'oléfines acycliques [articles de F. Pennella, dans "Journal of Catalysis", 69, 206 (1981) et T. Nishigushi, K. Fukuzumi et K. Sugisaki, dans "Journal of Catalysis", 70, 24 (1981)].

Or, au cours des travaux, qui ont conduit à la présente invention, on a découvert qu'à l'aide de catalyseurs appropriés, il est possible d'effectuer la métathèse d'oléfines acycliques et cycliques contenant du soufre et d'accéder ainsi à toute une gamme de produits soufrés oléfiniques, polyéniques et/ou polymères. Cette réaction peut permettre l'obtention économique de tels composés soufrés, en une seule étape, à partir de réactifs aisément disponibles.

Selon le nouveau procédé de l'invention, on soumet une oléfine acyclique ou un hydrocarbure cyclique insaturé non aromatique, contenant chacun du soufre dans leur chaîne hydrocarbonée, à la métathèse sur eux-mêmes, ou à la métathèse croisée avec une autre oléfine acyclique ou un autre hydrocarbure insaturé cyclique, non aromatiques ne contenant pas de soufre, de façon à produire un ou plusieurs composés porteurs d'une ou de plusieurs doubles liaisons et contenant un ou plusieurs atomes de soufre.

Le procédé que l'on préconise ici pour la préparation d'un composé organique ou d'un polymère qui comportent chacun (i) un ou plusieurs atomes de soufre sous forme sulfure, et (ii) une ou plusieurs doubles liaisons dans une chaîne hydrocarbonée, par métathèse d'un sulfure oléfinique, qui est cyclique ou acyclique, est caractérisé en ce que la métathèse est effectuée en présence d'un catalyseur constitué par un complexe du tungstène possédant au moins un groupe phenoxy et choisi parmi

(a) les composés de formule :

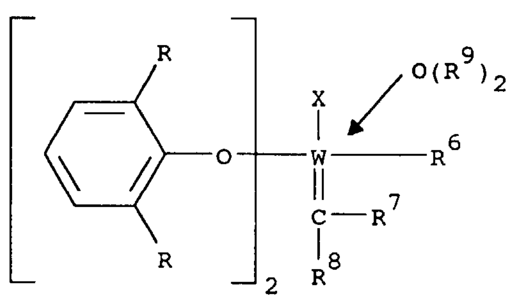

(15)

où X est un halogène, R un groupe hydrocarboné ou un groupe ou atome électronégatif, $R^6$ un alkyle, un aryle ou un halogène, $R^7$ et $R^8$ des atomes d'hydrogène ou des alkyles et $R^9$ un alkyle ;

(b) les composés de formule :

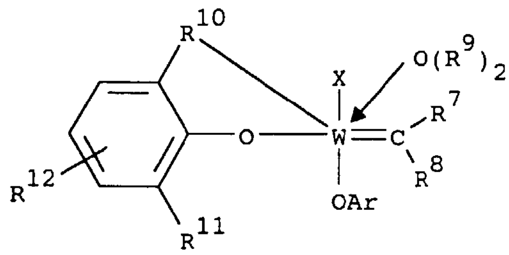

(16)

où $R^{11}$ et $R^{12}$ sont chacun H ou aryle, $R^{10}$ est un groupe divalent arylène, et Ar dans le groupe OAr lié au W est aryle, X et $R^7$ à $R^9$ étant définis comme ci-dessus ; et,

(c) les composés de formule :

$$WCl_{(6-x)}(OAr)_x \quad (17)$$

où OAr désigne un groupe aryloxy, x est 2, 3 ou 4, chaque composé de formule 17 étant associé à un composé organométallique de Ga, Al, Sn, Pb, Mg, Li, Ti ou B.

Des oléfines soufrées, acycliques, convenant à la réalisation du procédé selon l'invention, peuvent être choisies parmi de nombreux hydrocarbures non saturés, comportant au moins 3 atomes de carbone, une double liaison et un atome de S ; le procédé peut s'appliquer aux divers composés dont le nombre de C peut aller jusqu'à 36 et au delà ; celui des doubles liaisons à 4 et même plus, 1 à 8 atomes de S étant en général présents dans la molécule.

Dans les applications les plus courantes de l'invention, les sulfures oléfiniques, traitées, répondent à la formule

$$R^1\text{-CH=CH-}(CH_2)_n\text{-S-}R^2 \quad (1)$$

où $R^1$ et $R^2$ semblables ou différents, sont des atomes de H, des alkyles en $C_1$ à $C_{12}$ ou des cycloalkyles, des alcényles en $C_2$-$C_{12}$, ou des aryles en $C_6$ à $C_{18}$, n étant un nombre entier de 0 à 15.

Comme autres matières de départ du nouveau procédé, on peut utiliser des hydrocarbures cycliques insaturés, comportant de 1 à 4 doubles liaisons et possédant un groupement thioalkyle. En particulier, ce sont des oléfines cycliques soufrées répondant à la formule

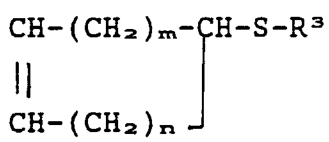

(2)

où m et n sont des nombres entiers, égaux ou différents, de 0 à 15, avec m+n différent de 0 et différent de 3, et $R^3$ est un alkyle ou alcényle en $C_1$ à $C_{12}$ ou un aryle de $C_6$-$C_{18}$. De tels produits de départ peuvent être facilement préparés par réaction d'addition d'un thiol à un diène cyclique, selon la méthode décrite par exemple dans l'article de K. Griesbaum, paru dans "Angewandte Chemie, International Edition in English", 9, 273 (1970).

Ces hydrocarbures insaturés acycliques ou cycliques non aromatiques, soufrés, matières de départ du nouveau procédé, peuvent être soumis à une réaction de métathèse sur eux-mêmes ou bien à la métathèse croisée avec un autre hydrocarbure insaturé acyclique ou cyclique, ne contenant pas de soufre.

Comme hydrocarbures insaturés acycliques conviennent par exemple des oléfines du type

$$R^4\text{-CH=CH-}R^5 \quad (3)$$

où $R^4$ et $R^5$, égaux ou différents, sont des atomes d'hydrogène, des alkyles en $C_1$ à $C_{18}$, des alcényles en $C_2$ à $C_{18}$ ou des aryles en $C_6$-$C_{18}$.

Les hydrocarbures insaturés cycliques possèdent le plus souvent de 1 à 4 doubles liaisons, et peuvent être représentés par la formule générale

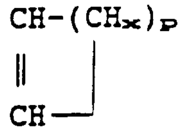

(4)

où x est égal à 1 ou 2 et p est un nombre entier égal à 2, 3 ou de 5 à 15 ; le cycle pouvant être multiple et comporter un pont. Des exemples préférés sont les cyclopentène, cyclooctène, cyclodécène, cyclododécène, cyclooctadiène, cyclododécadiène, cyclododécatriène ou cyclooctatétraène, oléfines ou diènes polycycliques, tels que norbornène, norbornadiène ou dicyclopentadiène, etc.

Un premier mode d'application du procédé selon l'invention consiste à faire réagir, par métathèse, sur elle-même l'oléfine acyclique soufrée (1) pour obtenir l'oléfine acyclique contenant 2 atomes de soufre de formule générale

$$R^2\text{-S-}(CH_2)_n\text{-CH=CH-}(CH_2)_n\text{-S-}R^2 \quad (5)$$

Une deuxième forme d'exécution consiste à faire réagir, par métathèse, sur lui-même l'hydrocarbure insaturé cyclique soufré (2) pour obtenir le polymère insaturé acyclique soufré, dont le motif répété est

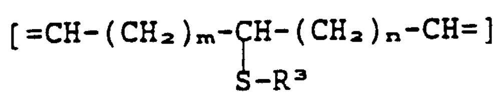

(6)

Dans une troisième réalisation, on fait réagir, par métathèse croisée, l'oléfine acyclique soufrée (1) avec l'oléfine acyclique (3), pour obtenir les nouvelles oléfines acycliques soufrées de formules

$$R^4\text{-CH=CH-}(CH_2)_n\text{-S-}R^2 \quad (7)$$

et

$$R^5\text{-CH=CH-}(CH_2)_n\text{-S-}R^2 \quad (8)$$

Une quatrième méthode réside en la métathèse croisée d'une oléfine acyclique soufrée (1) avec un hydrocarbure insaturé cyclique (4) pour former les diènes acycliques soufrés de formules

$$R^1\text{-CH=CH-}(CH_x)_p\text{-CH=CH-}(CH_2)_n\text{-S-}R^2 \quad (9)$$

et

$$R^2\text{-S-}(CH_2)_n\text{-CH=CH-}(CH_x)_p\text{-CH=CH-}(CH_2)_n\text{-S-}R^2 \quad (10)$$

Selon une cinquième forme d'exécution on fait réagir, par métathèse croisée, l'hydrocarbure insaturé cyclique soufré (2) avec l'oléfine acyclique (3) pour produire les diènes acycliques soufrés suivants :

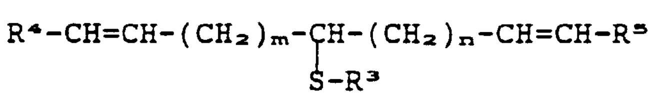

(11)

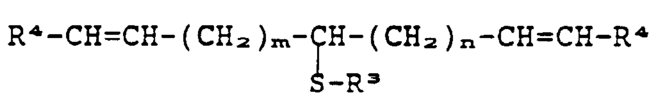

(12)

et

$$R^5-CH=CH-(CH_2)_m-\underset{\displaystyle S-R^3}{\underset{|}{C}}H-(CH_2)_n-CH=CH-R^5 \qquad (13)$$

Un sixième mode consiste à faire réagir, par métathèse croisée, l'hydrocarbure insaturé cyclique soufré (2) avec l'hydrocarbure insaturé cyclique (4), produisant ainsi le polymère insaturé acyclique soufré dont le motif répété est

$$[=CH-(CH_2)_m-\underset{\displaystyle S-R^3}{\underset{|}{C}}H-(CH_2)_n-CH=]_y[=CH-(CH_x)_p-CH=]_z \qquad (14)$$

où y et z identiques ou différents sont des nombres compris entre 300 et 2000 et surtout compris entre 500 et 1500.

La métathèse exige - comme on le sait - un catalyseur ; aussi les différents modes d'application du procédé selon l'invention, sont-ils réalisables en présence de catalyseurs connus dans l'art. On peut employer, par exemple, les catalyseurs indiqués dans les publications de FR-A-2 547 513, FR-A-2 577 216 (EP-A- 0191675) et FR-A-2 612 422. (EP-A- 0 283 400).

Des catalyseurs à base de tungstène sont surtout recommandables, en particulier des catalyseurs constitués par des complexes diphénoxy carbéniques du tungstène, de forme générale :

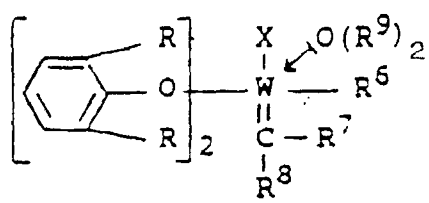

(15)

où X est un halogène, R un groupe hydrocarboné ou un groupe ou atome électronégatif, $R^6$ un alkyle, un aryle ou un halogène, $R^7$ et $R^8$ des atomes d'hydrogène ou des alkyles et $R^9$ un alkyle. On peut trouver la méthode de préparation et la description de tels catalyseurs dans la publication de brevet français 2 577 216 (EP-A- 0 191 675).

En particulier, X est un atome de chlore, les R sont des alkyles de $C_1$ à $C_6$, des aryles ou des atomes de Cl, Br ou F, $R^6$ un groupe neopentyle $-CH_2-C(CH_3)_3$ ou un atome de chlore, $R^7$ un groupe tertiobutyle $-C(CH_3)_3$, $R^8$ un atome d'hydrogène et $R^9$ un groupe éthyle $-CH_2CH_3$ ou un groupe isopropyle $-CH(CH_3)_2$.

Cependant, de façon tout à fait imprévue, il s'est avéré que les résultats de la métathèse, impliquant des composés au soufre, peuvent être considérablement améliorés par l'emploi de certains catalyseurs représentant une variante de ceux du type (15), décrits plus haut. Ces nouveaux catalyseurs au tungstène sont caractérisés en ce que leurs groupes R sont obligatoirement des aryles dont un est lié directement à l'atome de w. Ils peuvent être représentés par la structure suivante :

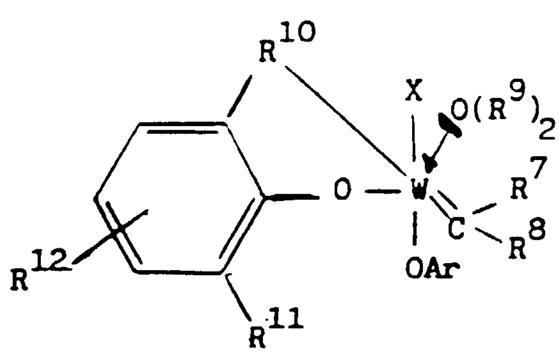

(16)

Dans ce complexe, les groupes $R^{10}$, $R^{11}$ et/ou $R^{12}$ sont des aryles, $R^{11}$ et $R^{12}$ pouvant ne pas exister ; ce ou ces

derniers, semblables ou différents, sont par exemple, des groupes phényle, tolyle, xylyle, mésityle, naphtyle ou autres. $R^{10}$, étant lié à l'atome de tungstène hexavalent, ainsi qu'au noyau du groupe aryloxy, peut être par exemple un radical phénylényle, tolylényle, xylylényle, mésitylényle, etc. En outre, W porte un second aryloxy (OAr) dont le groupe aryle peut être choisi parmi ceux qui ont été cités plus haut.

En ce qui concerne les radicaux $R^7$ à $R^9$, ils ont été définis à propos des catalyseurs 15.

Dans la publication FR-A-2 577 216 précitée, on a décrit la préparation des catalyseurs 15 au moyen de l'exemple 1 : sur 1 mole d'un précurseur $Cl_4W\text{-}[O\text{-}Ar]_2$ on fait agir 1,5 mol d'un composé organo-magnésien $MgR_2$ (R = alkyle), dans une solution éthérée. Si l'aryloxy du précurseur porte au moins un substituant arylique en ortho de l'atome d'oxygène, et que seulement 1 mole de $MgR_2$ soit employée, le complexe obtenu présente la structure 16 indiquée ci-dessus, qui donne des résultats grandement améliorés en métathèse d'oléfines en général.

Il est tout à fait remarquable que des modifications relativement légères dans la composition et la préparation du catalyseur apportent un résultat industriel très sensible ; cela dénote une importante activité inventive, dont on peut se rendre compte, en particulier à la lumière de l'exemple 1, dans la suite de la présente description.

Les catalyseurs préférés (16) peuvent également être préparés aisément, par réaction, dans du diétyl-éther, entre le composé alkylidyne du tungstène $W(CC(CH_3)_3)Cl_3$(diméthoxyéthane) [dont la préparation est décrite dans l'article de R.R. Schrock, J. Sancho et S.F. Pedersen paru dans "Inorganic Syntheses", 26, 44 (1989)], et du 2,6-diphénylphénate de lithium.

Les catalyseurs de métathèse de formule générale (15) ou (16) peuvent s'employer seuls, mais ils peuvent également être utilisés en association avec un co-catalyseur acide de Lewis, tel qu'un composé organométallique ou un halogénure de Ga, Al, Sn, Pb, Mg, Li, Ti ou B.

La réaction de métathèse est effectuée en absence de solvant ou dans un solvant qui peut être un hydrocarbure aromatique ou saturé. Des solvants préférés sont les chlorobenzène, bromobenzène, benzène ou toluène.

La réaction de métathèse peut être réalisée à une température de 0°C à 100°C, mais préférablement entre 20°C et 80°C.

Les rapports molaires hydrocarbures acycliques ou cycliques insaturés soufrés/catalyseur à base de tungstène sont en général compris entre 10 et 10000, de préférence entre 10 et 500 pour les composés soufrés acycliques et entre 100 et 1000 pour les composés soufrés cycliques. Dans le cas des réactions de métathèse croisée, les meilleurs rapports molaires hydrocarbure insaturé non soufré/hydrocarbure insaturé soufré sont compris entre 0,2 et 10.

L'invention est illustrée par les exemples non limitatifs qui suivent. Sauf autre indication, le catalyseur est celui de la formule (16) dans laquelle : X est Cl, $R^7$ est tertiobutyle $-C(CH_3)_3$, $R^8$ est H, $R^9$ est éthyle, $R^{10}$ est phénylène, $R^{11}$ est phényle, $R^{12}$ n'existe pas, tandis que OAr désigne un diphényl-2,6-phénoxy-1.

**Exemple 1**

**Metathèse de l'allyl-méthyl-sulfure sur lui-même.**

Cette réaction conduit à l'éthylène et à une oléfine acyclique disulfurée, selon l'équation chimique suivante :

$$2\ CH_2{=}CH\text{-}CH_2\text{-}S\text{-}CH_3 \rightarrow CH_2{=}CH_2 + CH_3\text{-}S\text{-}CH_2\text{-}CH{=}CH\text{-}CH_2\text{-}S\text{-}CH_3$$

Dans un réacteur pour travail discontinu, préalablement purgé à l'argon, on introduit d'abord 22 mg, soit $2{,}6 \times 10^{-5}$ mol, de catalyseur (16), puis 5 ml de chlorobenzène servant de solvant. Le réacteur est mis sous agitation et chauffé à 80°C. Ensuite, on introduit 0,056 ml, soit $5{,}2 \times 10^{-4}$ mol, d'allyl-méthyl-sulfure. Après un certain temps de réaction, on analyse la phase liquide et la phase gazeuse et on détermine la conversion de l'allyl-méthyl-sulfure et le rendement en éthylène et en oléfine di-sulfurée, qui est identifiée et caractérisée par couplage chromatographie phase gazeuse/spectrométrie de masse (M = 148). Après 10 heures de réaction, la conversion de l'allyl-méthyl-sulfure s'établit à 40 % et les rendements en éthylène et en oléfine disulfurée sont les mêmes et égaux à 20 % .

Des opérations identiques avec un catalyseur dont les $R^{10}$ et $R^{11}$ étaient des tertiobutyles, ont demandé 20 heures pour une conversion de 10 % et un rendement en éthylène de 5 % .

**Exemples 2 à 17**

**Métathèse d'alkyl-thio-cyclooctènes sur eux-mêmes**

Cette réaction conduit à des polymères oléfiniques soufrés selon l'équation chimique suivante :

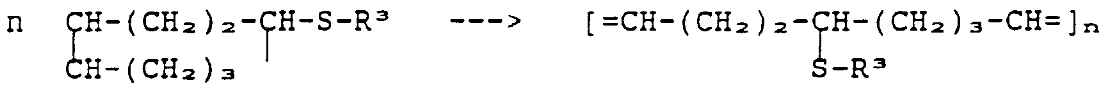

Dans un réacteur pour travail discontinu, préalablement purgé à l'argon, on introduit d'abord 22 mg, soit 2,6 x $10^{-5}$ mol, de catalyseur (16), puis l'alkyl-thio-cyclooctène, soit pur, soit en solution dans 5 ml de chlorobenzène. La formule générale des alkyl-thio-cyclooctènes utilisés est donnée dans l'équation chimique ci-dessus, la nature des groupes $R^3$ étant précisée dans les tableaux 1 et 2 ci-après. La quantité d'alkyl-thio-cyclooctène introduite est également précisée dans les tableaux 1 et 2 et exprimée par le rapport molaire alkyl-thio-cyclooctène/catalyseur. Le réacteur est mis sous agitation et la réaction de polymérisation est conduite soit à 20°C, soit à 80°C. Dans le cas où la polymérisation est conduite en présence de solvant, on analyse, après un temps de réaction précisé dans le tableau 1, la conversion de l'alkyl-thio-cyclooctène de départ. Dans le cas où la polymérisation est conduite en absence de solvant, on laisse la réaction se poursuivre pendant un certain temps, indiqué dans le tableau 2, puis le polymère formé est récupéré et pesé, ce qui permet de déterminer le rendement de la réaction, exprimé en poids de polymère obtenu par rapport au poids d'alkyl-thio-cyclooctène introduit. Les résultats sont donnés dans le tableau 2. La structure des polymères obtenus, dont la formule générale est indiquée dans l'équation chimique ci-dessus, est vérifiée par analyse chimique élémentaire et par analyse de leurs spectres de résonance magnétique nucléaire du proton et du carbone 13.

**TABLEAU 1**

Polymérisation par métathèse, en présence de solvant, des alkyl-thio-cyclooctènes de formule générale :

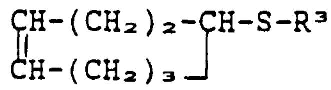

| Ex.No | $R^3$ | Température de réaction | (2)/W | Temps de réaction | Conversion |
|---|---|---|---|---|---|
| 2 | Et | 20°C | 100 | 200 min | 97 % |
| 3 | n-Bu | 20°C | 100 | 120 min | 97 % |
| 4 | t-Bu | 20°C | 100 | 10 min | 99 % |
| 5 | Hex | 20°C | 100 | 100 min | 99 % |
| 6 | cyclo-Hex | 20°C | 100 | 30 min | 97 % |
| 7 | Et | 80°C | 100 | 10 min | 97 % |
| 8 | n-Bu | 80°C | 100 | 10 min | 97 % |
| 9 | t-Bu | 80°C | 500 | 30 min | 96 % |
| 10 | cyclo-Hex | 80°C | 500 | 60 min | 83 % |

(2)/W = rapport molaire initial alkyl-thio-cyclooctène/catalyseur.
Conversion = alkyl-thio-cyclooctène converti / alkyl-thio-cyclooctène initial.

**TABLEAU 2**

Polymérisation par métathèse, en absence de solvant, des alkyl-thio-cyclooctènes de formule générale

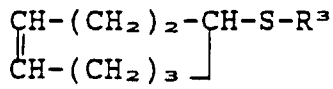

| Ex No | $R^3$ | Température de réaction | (2)/W | Temps de réaction | Rendement |
|---|---|---|---|---|---|
| 11 | Et | 20°C | 100 | 2,5 min | 65 % |
| 12 | n-Bu | 20°C | 100 | 2 min | 67 % |
| 13 | t-Bu | 20°C | 100 | 2 min | 65 % |
| 14 | Hex | 20°C | 100 | 3,5 min | 62 % |
| 15 | cyclo-Hex | 20°C | 100 | 2 min | 53 % |
| 16 | Et | 20°C | 500 | 20 min | 51 % |
| 17 | n-Bu | 20°C | 500 | 30 min | 46 % |

(2)/W = rapport molaire initial alkyl-thio-cyclooctène / catalyseur.
Rendement = poids de polymère obtenu/poids d'alkyl-thio-cyclooctène initial.

Les polymères présentaient les masses moléculaires en poids Mp et les températures de transition vitreuse $T_G$ suivantes :

| Exemple No | Mp | $T_G$ |
|---|---|---|
| 11 | 119 400 | -68°C |
| 12 | 150 900 | -72°C |
| 13 | 140 100 | -26°C |
| 14 | 153 100 | - |
| 15 | 181 800 | - |
| 16 | 176 600 | -74°C |
| 17 | 196 500 | -80°C |

**Exemples 18 et 19**

**Métathèse croisée de l'allyl-méthyl-sulfure avec une oléfine acyclique non soufrée.**

Cette réaction conduit à la formation de nouvelles oléfines soufrées selon l'équation chimique suivante:

$$2\ CH_2{=}CH{-}CH_2{-}S{-}CH_3 + 2\ R^4{-}CH{=}CH{-}R^5 \rightarrow R^4{-}CH{=}CH{-}CH_2{-}S{-}CH_3$$

$$+\ R^5{-}CH{=}CH{-}CH_2{-}S{-}CH_3$$

$$+\ R^4{-}CH{=}CH_2$$

$$+\ R^5{-}CH{=}CH_2$$

Dans un réacteur pour travail discontinu, préalablement purgé à l'argon, on introduit d'abord 22 mg, soit 2,6 x $10^{-5}$ mol, de catalyseur (16), puis 5 ml de chlorobenzène servant de solvant. Ensuite, on introduit l'oléfine acyclique non soufrée de formule générale $R^4$-CH=CH-$R^5$ en une quantité donnée dans le tableau 3 (et exprimée en rapport molaire par rapport au catalyseur) et l'allyl-méthyl-sulfure en une quantité également donnée dans le tableau 3. Le réacteur est mis sous agitation et laissé à 20°C ou chauffé à 80°C. Après un certain temps de réaction, on analyse le mélange réactionnel et on détermine les rendements en produits soufrés de formules $R^4$-CH=CH-$CH_2$-S-$CH_3$ et $R^5$-CH=CH-$CH_2$-S-$CH_3$ qui sont caractérisés et identifiés par couplage chromatographie phase gazeuse - spectrométrie de masse. Les résultats sont donnés dans le tableau 3.

TABLEAU 3

| Métathèse croisée entre l'allyl-méthyl-sulfure (1) et une oléfine acyclique de formule $R^4$-CH=CH-$R^5$ (3). | | |
|---|---|---|
| Ex No | 18 | 19 |
| $R^4$ | -$CH_3$ | -$CH_3$ |
| $R^5$ | -$CH_3$ | -$CH_2CH_3$ |
| (1)/W | 25 | 20 |
| (3)/W | 100 | 20 |
| Température de réaction | 20°C | 80°C |
| Temps de réaction | 15 h | 15 h |
| Rendement en $R^4$-CH=CH-$CH_2$-S-$CH_3$ | 95 % | 30 % |
| Rendement en $R^5$-CH=CH-$CH_2$-S-$CH_3$ | - | 20 % |
| (1)/W ou (3)/W = rapport molaire initial oléfine/ catalyseur. Les rendements en produits sont exprimés en nombre de moles de produit obtenu par rapport au nombre de moles d'allyl-méthyl-sulfure introduit. | | |

**Exemples 20 à 22**

**Métathèse croisée de l'allyl-méthyl-sulfure avec une oléfine cyclique non soufrée.**

Cette réaction conduit à la formation de diènes soufrés selon l'équation chimique suivante :

$$3\ CH_2{=}CH{-}CH_2{-}S{-}CH_3\ +\ 3$$ 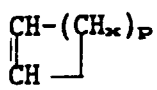

$$\text{--->}\ CH_2{=}CH{-}(CH_x)_p{-}CH{=}CH{-}CH_2{-}S{-}CH_3$$

$$+\ CH_3{-}S{-}CH_2{-}CH{=}CH{-}(CH_x)_p{-}CH{=}CH{-}CH_2{-}S{-}CH_3$$

$$+\ CH_2{=}CH{-}(CH_x)_p{-}CH{=}CH_2$$

Dans un réacteur pour travail discontinu, préalablement purgé à l'argon, on introduit d'abord 22 mg, soit 2,6 x $10^{-5}$ mol, de catalyseur(16), puis 5 ml de chlorobenzène servant de solvant. Le réacteur est mis sous agitation et laissé à 20°C ou chauffé à 80°C. Ensuite on introduit l'allyl-méthyl-sulfure en une quantité précisée dans le tableau 4 et l'oléfine cyclique non soufrée qui est soit le cyclopentène (exemple 20), soit le norbornène (exemples 21 et 22) en une quantité également précisée dans le tableau 4. Après un certain temps de réaction, on analyse la phase liquide et on détermine la conversion de l'allyl-méthyl-sulfure et de l'oléfine cyclique. Les résultats sont donnés dans le tableau 4. Les principaux produits formés sont les diènes dont la formule générale se trouve dans l'équation chimique ci-dessus ; ils sont identifiés

et caractérisés par couplage chromatographie phase gazeuse - spectrométrie de masse. Ces diènes ont respectivement des masses molaires de 156, 216 et 96 (dans le cas de la métathèse croisée avec le cyclopentène), et de 182, 242 et 122 (dans le cas de la métathèse croisée avec le norbornène).

TABLEAU 4

| Métathèse croisée entre l'allyl-méthyl-sulfure (1) et le cyclopentène ou le norbornène. | | | |
|---|---|---|---|
| Ex No | 20 | 21 | 22 |
| (1)/W | 25 | 25 | 25 |
| cyclopentène/W | 25 | - | - |
| norbornène/W | - | 25 | 100 |
| Température de réaction | 80°C | 20°C | 20°C |
| Temps de réaction | 3 h | 30 min | 20 min |
| Conversion de (1) | 30 % | 13 % | 32 % |
| Conversion de l'oléfine cyclique | 27 % | 99 % | 99 % |
| (1)/W ou oléfine cyclique/W = rapport molaire initial oléfine/catalyseur. | | | |

**Exemples 23 à 32**

**Métathèse croisée entre le butyl-thio-cyclooctène et une oléfine acyclique non soufrée.**

Cette réaction conduit à la formation de diènes acycliques soufrés selon l'équation chimique suivante :

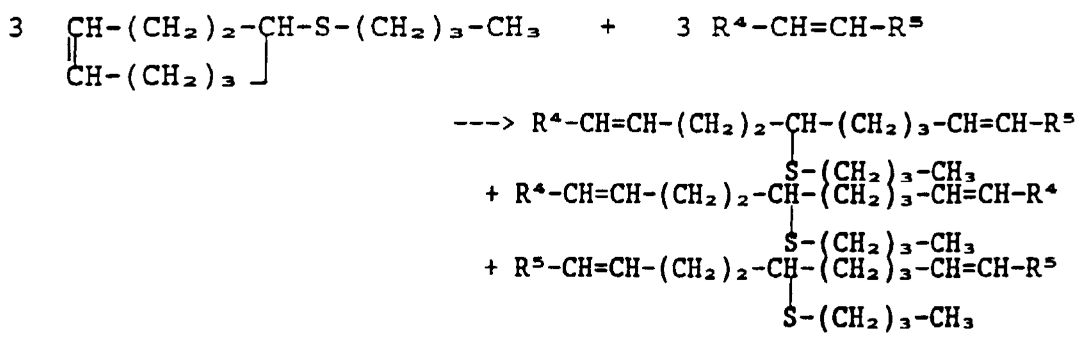

Dans un réacteur pour travail discontinu, préalablement purgé à l'argon, on introduit d'abord 22 mg, soit 2,6 x $10^{-5}$ mol, de catalyseur (16), puis 5 ml de chlorobenzène servant de solvant. Le réacteur est mis sous agitation et laissé à 20°C ou chauffé à 80°C. Ensuite, on introduit le butyl-thio-cyclooctène en une quantité qui est précisée dans le tableau 5 et l'oléfine acyclique (3) en une quantité également précisée dans le tableau 5. Après un certain temps de réaction, on analyse la phase liquide et on détermine la conversion du butyl-thio-cyclooctène et le rendement en diènes soufrés dont les formules générales apparaissent dans l'équation chimique ci-dessus. Ces diènes soufrés sont identifiés et caractérisés par couplage chromatographie phase gazeuse - spectrométrie de masse.

TABLEAU 5

| Métathèse croisée entre le butyl-thio-cyclooctène (2) et une oléfine acyclique non soufrée $R^4$-CH=CH-$R^5$ (3). | | | | | | | | |
|---|---|---|---|---|---|---|---|---|
| Ex. No | $R^4$ | $R^5$ | (2)/W | (3)/W | T | t | Conv. | Rend. |
| 23 | H | H | 25 | 200 | 20°C | 6 h | 99 % | 75 % |
| 24 | H | H | 50 | 200 | 20°C | 15 h | 99 % | 72 % |
| 25 | H | H | 50 | 400 | 20°C | 16 h | 88 % | 71 % |
| 26 | H | H | 100 | 200 | 20°C | 17 h | 96 % | 59 % |
| 27 | H | H | 200 | 200 | 20°C | 8 h | 82 % | 39 % |
| 28 | H | H | 50 | 200 | 80°C | 15 min | 93 % | 61 % |
| 29 | H | H | 50 | 400 | 80°C | 12 min | 95 % | 64 % |
| 30 | H | H | 100 | 200 | 80°C | 10 min | 96 % | 33 % |

TABLEAU 5 (suite)

| Métathèse croisée entre le butyl-thio-cyclooctène (2) et une oléfine acyclique non soufrée $R^4$-CH=CH-$R^5$ (3). | | | | | | | | |
|---|---|---|---|---|---|---|---|---|
| Ex. No | $R^4$ | $R^5$ | (2)/W | (3)/W | T | t | Conv. | Rend. |
| 31 | H | $CH_3$ | 50 | 200 | 80°C | 15 min | 97 % | 55 % |
| 32 | $CH_3$ | $CH_3$ | 50 | 200 | 80°C | 15 min | 94 % | 52 % |

(2)/W et (3)/W = rapports molaires initiaux butyl-thiocyclooctène/catalyseur et oléfine acyclique/catalyseur.
T = Température de réaction.
t = Temps de réaction.
Conv. = Conversion du butyl-thio-cyclooctène.
Rend. = Rendement en diènes soufrés = nombre de moles de diènes soufrés obtenu/nombre de moles de butyl-thio-cyclooctène initial.

Exemples 33 à 41

Métathèse croisée entre le butyl-thio-cyclooctène et un hydrocarbure insaturé cyclique non soufré.

Cette réaction conduit à la formation de polymères oléfiniques soufrés selon l'équation chimique suivante :

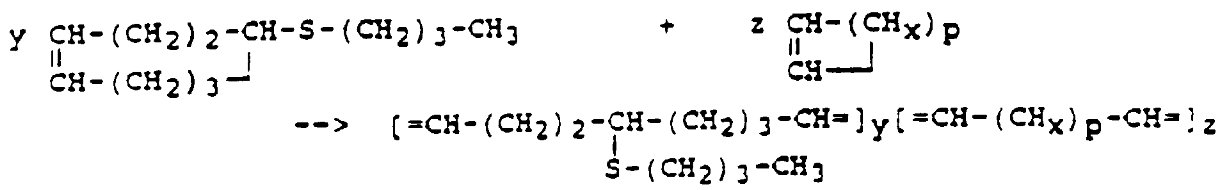

Dans un réacteur pour travail discontinu, préalablement purgé à l'argon, on introduit d'abord 22 mg, soit 2,6 x $10^{-5}$ mol, de catalyseur (16), puis le butyl-thio-cyclooctène et l'hydrocarbure cyclique insaturé non soufré en des quantités qui sont précisées dans le tableau 6. Le réacteur est mis sous agitation et laissé à température ambiante. Après un certain temps de réaction, le polymère formé est récupéré et pesé. Le rendement de la réaction de polymérisation est exprimé par le rapport entre le poids de polymère obtenu et la somme des poids des hydrocarbures cycliques soufrés et non soufrés introduits. Les polymères sont caractérisés par analyse chimique élémentaire et par analyse de leurs spectres de résonance magnétique nucléaire du proton et du carbone 13.

TABLEAU 6

| Métathèse croisée entre le butyl-thio-cyclooctène (2) et un hydrocarbure cyclique insaturé non soufré (4) | | | | | |
|---|---|---|---|---|---|
| Ex. No | (4) | (2)/W | (4)/W | Temps de réaction | Rend. |
| 33 | norbornène | 500 | 500 | 2 min | 60 % |
| 34 | norbornène | 100 | 30 | 1 min | 75 % |
| 35 | cyclopentène | 100 | 100 | 20 min | 55 % |
| 36 | cyclooctène | 100 | 100 | 30 s | 80 % |
| 37 | cyclooctadiène | 100 | 100 | 1 min | 70 % |
| 38 | cyclododécène | 100 | 100 | 4 min | 55 % |
| 39 | cyclododécatriène | 100 | 100 | 3 min | 70 % |
| 40 | norbornadiène | 100 | 100 | 30 s | 30 % |
| 41 | dicyclopentadiène | 100 | 100 | 2 min | 45 % |

(2)/W et (4)/W = rapports molaires initiaux butyl-thiocyclooctène/catalyseur et hydrocarbure insaturé cyclique non soufré/catalyseur.

Rendement = poids de polymère obtenu/somme des poids de butyl-thio-cyclooctène et d'hydrocarbure insaturé cyclique non soufré introduits.

Les polymères solides, obtenus, présentaient les caractéristiques suivantes :

| Exemple No | Mp | $T_G$ | y/z |
|---|---|---|---|
| 33 | 410 000 | 59°C | 0,16 |
| 34 | 168 400 | -69°C | 2,70 |
| 35 | 80 740 | -82°C | 0,75 |
| 36 | 276 300 | -80°C | 1,04 |
| 37 | 165 600 | -84°C | 1,00 |
| 38 | 403 100 | -76°C | 3,00 |
| 39 | 484 400 | -75°C | 1,38 |
| 40 | - | -20°C | - |
| 41 | 385 400 | 58°C | 0,15 |

Le rapport y/z exprime le rapport entre le nombre d'unités monomères contenant du soufre et le nombre d'unités monomères ne contenant pas de soufre ; ces rapports ont été mesurés, dans les polymères obtenus, par analyse élémentaire et par analyse RMN.

**Exemples 42 à 46**

**Métathèse du sulfure d'allyle sur lui-même.**

Cette métathèse intramoléculaire du sulfure d'allyle, donne de l'éthylène et du 2,5-dihydrothiophène, selon l'équation chimique :

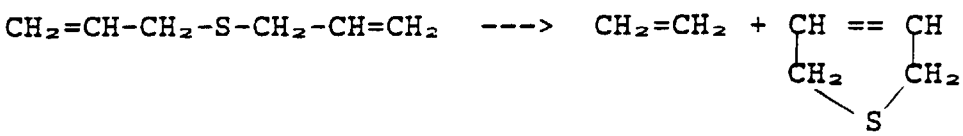

Dans un réacteur pour travail discontinu, préalablement purgé à l'argon, on introduit 22 mg, soit 2,6 x $10^{-5}$ mol, de catalyseur (16). On introduit ensuite le sulfure d'allyle, soit pur, soit en solution dans 5 ml de chlorobenzène, en une quantité qui est précisée dans le tableau des résultats ci-après et exprimée par le rapport molaire sulfure d'allyle/catalyseur. Le réacteur est mis sous agitation et chauffé à 50°C ou 80°C.

Après un certain temps de réaction, on analyse la phase liquide et on détermine la conversion du sulfure d'allyle et le rendement en 2,5-dihydrothiophène. Le 2,5-dihydrothiophène est caractérisé par spectrométrie de masse (M = 86) et par ses spectres de résonance magnétique nucléaire du proton (100 MHz) et du carbone 13 (découplé proton) (25 MHz) dans le deutérochloroforme (déplacements chimiques en ppm par rapport à un étalon tétraméthylsilane) : RMN $^1H$ : 5,83 (2 groupes -CH=) ; 3,73 (2 groupes $-CH_2-$) ; RMN $^{13}C$: 128,4 (2 groupes -CH=) ; 38,8 (2 groupes $-CH_2-$).

TABLEAU 7

| Métathèse du sulfure d'allyle (S) conduisant au 2,5-dihydrothiophène (DHT). | | | | | | |
|---|---|---|---|---|---|---|
| Ex No | S/W | PhCl | Température de réaction | Temps de réaction | Conversion de S | Rendement en DHT |
| 42 | 100 | 5 ml | 50°C | 3 h | 98 % | 85 % |
| 43 | 100 | 0 | 80°C | 8 h | 99 % | 90 % |
| 44 | 250 | 5 ml | 50°C | 6 h | 88 % | 80 % |
| 45 | 250 | 5 ml | 80°C | 3 h | 98 % | 90 % |
| 46 | 500 | 5 ml | 80°C | 2 h | 58 % | 55 % |
| S/W = rapport molaire initial sulfure d'allyle /catalyseur. | | | | | | |

On voit qu'il est possible d'obtenir des dérivés du thiophène, avec un excellent rendement, par le procédé de l'invention.

En général, dans le cadre de la présente invention, la métathèse des oléfines cycliques seules donne des polymères acycliques ; par métathèse croisée avec des oléfines acycliques en proportion moléculaire du même ordre,

elles donnent un mélange de polymère et de nouvelle oléfine acyclique (en particulier de diène). Avec un excès d'oléfine acyclique, une oléfine cyclique peut donner une nouvelle oléfine non cyclique, par l'ouverture de son cycle. Ainsi, l'invention permet-elle de faire varier la nature des produits par le réglage des proportions des oléfines mises en oeuvre.

Les exemples, et la description qui les précède, montrent comment l'invention permet de préparer divers composés insaturés, porteurs d'atomes de soufre liés au carbone. Les principaux de ces produits nouveaux peuvent être représentés de la façon suivante.

Hydrocarbures insaturés, monomères, possédant au moins une fonction thioéther, du type

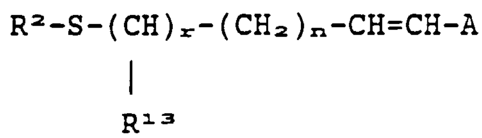

$R^2$ étant défini plus haut, à propos de la formule (1),

$R^{13}$ pouvant être choisi parmi les mêmes groupes qui forment $R^2$, r est 1 ou 0, et n = 0 à 15, tandis que A représente un des groupements tels que :

$R^4$- (voir formule 3)
$R^2S\ (CH_2)_n$- ;
$R^1$-CH=CH-$(CH_x)_p$-, $R^1$ étant décrit avec la formule (1) et pouvant être le même que $R^2$ ; X est 1 ou 2, et p = 2 ou 3, ou bien 5 à 15 ;
$R^4$-CH=CH-$(CH_2)_m$- avec m = 0 à 15 ou $R^1$-S-$(CH_2)_n$-CH=CH-$(CH_x)_p$-.

Quant aux polymères, on peut noter, en particulier, ceux dont la structure comprend le motif :

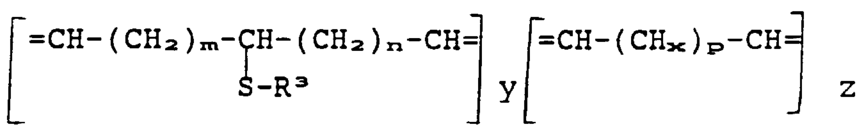

"y" étant en général 300 à 2 000 et "z" = 0 ou bien 300 à 2 000.

**Exemples comparatifs**

Les opérations de l'exemple 42 ont été répétées avec d'autres catalyseurs, dans les conditions suivantes : rapport molaire initial sulfure d'allyle/catalyseur = 100 ;

5 ml de chlorobenzène comme solvant ;
température de réaction 50°C ;
temps de réaction 3 heures.

Résultats

| | Catalyseur | Conversion de S | Rendement en DHT |
|---|---|---|---|
| 42 | (16) | 98 % | 85 % |
| 42a | Catalyseur "K" selon brevet européen 0191675 | 70 % | 61 % |
| 42b | Catalyseur $Re_2O_7/Al_2O_3$ | 10 % | 5 % |

**Exemples 47 et 48**

La métathèse du sulfure d'allyle en 2,5-dihydrothiophène (DHT) a été effectuée selon la technique des exemples 42-46 en 8 heures à 80°C avec 5 ml de chlorobenzène comme solvant, le catalyseur étant

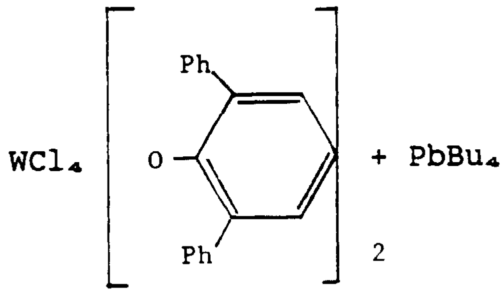

(décrit dans le brevet européen 0 129 474).

Les résultats suivants ont été obtenus :

| | Exemple 47 | Exemple 48 |
|---|---|---|
| Pb/W | 2 | 2 |
| S/W | 100 | 50 |
| Conversion de S | 50 % | 90 % |
| Rendement en DHT | 50 % | 90 % |

**Revendications**

**1.** Procédé de préparation d'un composé organique ou d'un polymère qui comportent chacun (i) un ou plusieurs atomes de soufre sous forme sulfure, et (ii) une ou plusieurs doubles liaisons dans une chaîne hydrocarbonée, par métathèse d'un sulfure oléfinique, qui est cyclique ou acyclique, caractérisé en ce que la métathèse est effectuée en présence d'un catalyseur constitué par un complexe du tungstène possédant au moins un groupe phenoxy et choisi parmi

(a) les composés de formule :

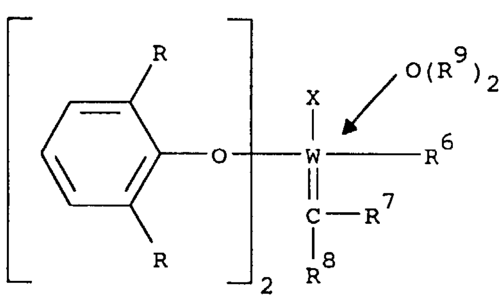

(15)

où X est un halogène, R un groupe hydrocarboné ou un groupe ou atome électronégatif, $R^6$ un alkyle, un aryle ou un halogène, $R^7$ et $R^8$ des atomes d'hydrogène ou des alkyles et $R^9$ un alkyle ;

(b) les composés de formule :

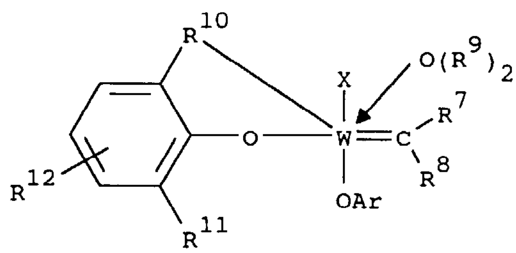

(16)

où $R^{11}$ et $R^{12}$ sont chacun H ou aryle, $R^{10}$ est un groupe divalent arylène, et Ar dans le groupe OAr lié au W

est aryle, X et $R^7$ à $R^9$ étant définis comme ci-dessus ; et,
(c) les composés de formule :

$$WCl_{(6-x)}(OAr)_x \qquad (17)$$

où OAr désigne un groupe aryloxy, x est 2, 3 ou 4, chaque composé de formule 17 étant associé à un composé organométallique de Ga, Al, Sn, Pb, Mg, Li, Ti ou B.

**2.** Procédé suivant la revendication 1, caractérisé en ce que le sulfure oléfinique est soumis à la métathèse croisée avec une oléfine acyclique ou un hydrocarbure insaturé cyclique et non aromatique, qui ne contiennent pas de soufre.

**3.** Procédé suivant la revendication 1, caractérisé en ce que le sulfure oléfinique est soumis à la métathèse sur lui-même.

**4.** Procédé suivant la revendication 2 ou 3, caractérisé en ce que le sulfure oléfinique est un composé acyclique qui répond à la formule :

$$R^1\text{-}CH = CH\text{-}(CH_2)_n\text{-}S\text{-}R^2$$

où $R^1$ et $R^2$, semblables ou différents, sont des atomes de H, des alkyles en $C_1$-$C_{12}$, des cycloalkyles, des alcényles en $C_2$ à $C_{12}$ ou des aryles en $C_6$ à $C_{18}$, n étant un nombre entier de 0 à 10.

**5.** Procédé suivant la revendication 4, caractérisé en ce que le sulfure oléfinique acyclique est l'allyle-méthyl sulfure.

**6.** Procédé suivant la revendication 4, caractérisé en ce que le sulfure oléfinique acyclique est le sulfure d'allyle.

**7.** Procédé suivant la revendication 2 ou 3, caractérisé en ce que le sulfure oléfinique est un composé cyclique, qui comporte de 1 à 4 doubles liaisons dans sa chaîne hydrocarbonée et possède un groupe thioalkyle.

**8.** Procédé suivant la revendication 7, caractérisé en ce que le sulfure oléfinique cyclique répond à la formule

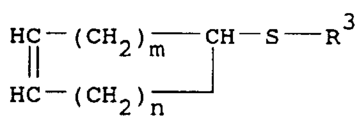

où m et n sont des nombres entiers, égaux ou différents, de 0 à 15, avec m+n différent de 0 et différent de 3, et $R^3$ est un alkyle en $C_1$ à $C_{12}$, un alcényle en $C_2$ à $C_{12}$, ou un aryle en $C_6$ à $C_{18}$.

**9.** Procédé suivant la revendication 8, caractérisé en ce que m est égal à 2, n égal à 3 et le groupe $R^3$ est un groupe éthyle, n-butyle, tertiobutyle, hexyle ou cyclo-hexyle.

**10.** Procédé suivant la revendication 2, caractérisé en ce que l'oléfine acyclique non soufrée, utilisée dans la réaction de métathèse croisée, répond à la formule $R^4$-CH=CH-$R^5$, où $R^4$ et $R^5$, semblables ou différents, sont des atomes d'hydrogène, des alkyles de $C_1$ à $C_{10}$, des alcényles en $C_2$-$C_{10}$ ou des aryles en $C_6$ à $C_{10}$.

**11.** Procédé suivant la revendication 10, caractérisé en ce que $R^4$ et $R^5$ sont simultanément ou indépendamment, H, $CH_3$ ou $CH_2$-$CH_3$.

**12.** Procédé suivant la revendication 2, caractérisé en ce que l'hydrocarbure cyclique insaturé et non soufré, utilisé dans les réactions de métathèse croisée, possède de 1 à 4 doubles liaisons et répond à la formule :

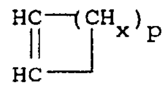

où x est égal à 1 ou 2 et p est un nombre entier égal à 2, 3 ou de 5 à 15, cet hydrocarbure pouvant être multicyclique et comporter un pont.

**13.** Procédé suivant la revendication 12, caractérisé en ce que l'hydrocarbure cyclique insaturé est une oléfine, un diène, un triène ou un tétraène cyclique.

**14.** Procédé suivant la revendication 13, caractérisé en ce que l'hydrocarbure cyclique insaturé est le cyclopentène, le cyclooctène, le cyclodécène, le cyclododécène, le cyclooctadiène, le cyclododécadiène, le cyclododécatriène ou le cyclooctatétraène.

**15.** Procédé suivant la revendication 12, caractérisé en ce que l'hydrocarbure cyclique insaturé est une oléfine ou un diène polycyclique.

**16.** Procédé suivant la revendication 15, caractérisé en ce que l'hydrocarbure insaturé polycyclique est le norbornène, le norbornadiène ou le dicyclopentandiène.

**17.** Procédé suivant la revendication 1, caractérisé en ce que le catalyseur de formule (15) ou (16) est associé à un halogénure ou à un composé organométallique de Ga, Al, Sn, Pb, Mg, Li, Ti ou B.

**18.** Procédé suivant la revendication 1, caractérisé en ce que l'on fait appel à un catalyseur de formule (17), qui est un complexe $WCl_4(OAr)_2$ associé à un composé organométallique de Pb ou de Sn.

**Patentansprüche**

**1.** Verfahren zur Herstellung einer organischen Verbindung oder eines Polymers, die jeweils i) ein oder mehrere Schwefelatome in Form von Sulfid und ii) eine oder mehrere Doppelbindungen in einer Kohlenwasserstoffkette aufweisen, durch Metathese eines Olefinsulfids, das zyklisch oder azyklisch ist, dadurch gekennzeichnet, daß die Metathese in Gegenwart eines Katalysators durchgeführt wird, der aus einem Wolframkomplex besteht, der mindestens eine Phenoxygruppe besitzt und ausgewählt ist aus:

a) den Verbindungen der Formel

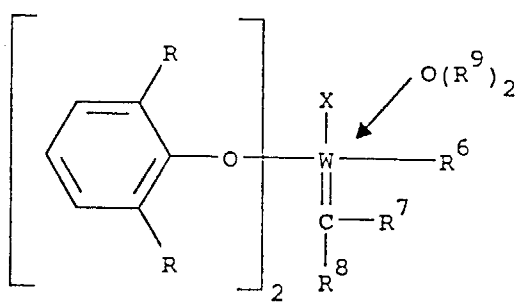

(15)

in der X ein Halogen, R eine Kohlenwasserstoffgruppe oder eine elektronegative Gruppe oder ein elektronegatives Atom ist, $R^6$ ein Alkyl, ein Aryl oder ein Halogen ist, $R^7$ und $R^8$ Wasserstoffatome oder Alkyle sind und $R^9$ ein Alkyl ist,

b) den Verbindungen der Formel

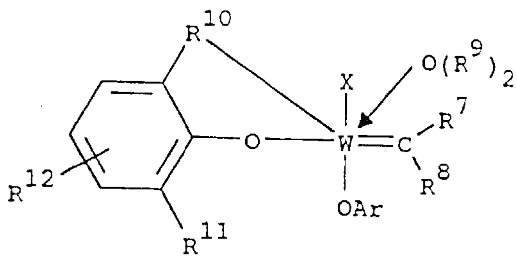

(16)

in der $R^{11}$ und $R^{12}$ jeweils H oder Aryl sind, $R^{10}$ eine zweiwertige Arylengruppe ist und Ar in der an das W gebundenen Gruppe OAr Aryl ist, wobei X und $R^7$ bis $R^9$ die oben genannten Bedeutungen haben, und
c) den Verbindungen der Formel

$$WCl_{(6-x)}(OAr)_x, \qquad (17)$$

in der OAr eine Aryloxygruppe ist und x für 2, 3 oder 4 steht, wobei jede Verbindung der Formel (17) an eine metallorganische Verbindung von Ga, Al, Sn, Pb, Mg, Li, Ti oder B gebunden ist.

**2.** Verfahren nach Anspruch 1, dadurch gekennzeichnet, daß das Olefinsulfid einer kreuzweisen Metathese mit einem azyklischen Olefin oder einem zyklischen und nichtaromatischen ungesättigten Kohlenwasserstoff, die keinen Schwefel enthalten, unterzogen wird.

**3.** Verfahren nach Anspruch 1, dadurch gekennzeichnet, daß das Olefinsulfid einer Metathese an sich selbst unterzogen wird.

**4.** Verfahren nach Anspruch 2 oder 3, dadurch gekennzeichnet, daß das Olefinsulfid eine azyklische Verbindung ist, die der Formel

$$R^1\text{-CH=CH-}(CH_2)_n\text{-S-}R^2$$

entspricht, in der $R^1$ und $R^2$, die gleich oder verschieden sind, H-Atome, $C_1$-$C_{12}$-Alkyl, Cycloalkyl, $C_2$-$C_{12}$-Alkenyl oder $C_6$-$C_{18}$-Aryl sind, wobei n eine ganze Zahl von 0 bis 10 ist.

**5.** Verfahren nach Anspruch 4, dadurch gekennzeichnet, daß das azyklische Olefinsulfid Allyl-methyl-sulfid ist.

**6.** Verfahren nach Anspruch 4, dadurch gekennzeichnet, daß das azyklische Olefinsulfid Allylsulfid ist.

**7.** Verfahren nach Anspruch 2 oder 3, dadurch gekennzeichnet, daß das Olefinsulfid eine zyklische Verbindung ist, die 1 bis 4 Doppelbindungen in ihrer Kohlenwasserstoffkette aufweist und eine Thioalkylgruppe besitzt.

**8.** Verfahren nach Anspruch 7, dadurch gekennzeichnet, daß das zyklische Olefinsulfid der Formel

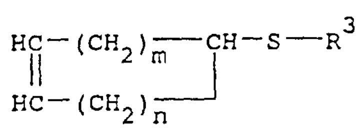

entspricht, in der m und n gleiche oder verschiedene ganze Zahlen von 0 bis 15 sind, wobei m+n nicht 0 und nicht 3 ist, und $R^3$ ein $C_1$-$C_{12}$-Alkyl, ein $C_2$-$C_{12}$-Alkenyl oder ein $C_6$-$C_{18}$-Aryl ist.

**9.** Verfahren nach Anspruch 8, dadurch gekennzeichnet, daß m gleich 2 ist, n gleich 3 ist und die Gruppe $R^3$ eine Ethyl-, n-Butyl-, tert.-Butyl-, Hexyl- oder Cyclohexyl-Gruppe ist.

**10.** Verfahren nach Anspruch 2, dadurch gekennzeichnet, daß das in der kreuzweisen Metathesereaktion verwendete azyklische nichtschwefelhaltige Olefin der Formel $R^4$-CH=CH-$R^5$ entspricht, in der $R^4$ und $R^5$, die gleich oder verschieden sind, Wasserstoffatome, $C_1$-$C_{10}$-Alkyl, $C_2$-$C_{10}$-Alkenyl oder $C_6$-$C_{10}$-Aryl sind.

**11.** Verfahren nach Anspruch 10, dadurch gekennzeichnet, daß $R^4$ und $R^5$ gleichzeitig oder unabhängig H, $CH_3$ oder $CH_2$-$CH_3$ sind.

**12.** Verfahren nach Anspruch 2, dadurch gekennzeichnet, daß der in den kreuzweisen Metathesereaktionen verwendete zyklische ungesättigte und nichtschwefelhaltige Kohlenwasserstoff 1 bis 4 Doppelbindungen besitzt und der Formel

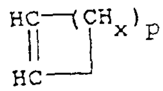

entspricht, in der x gleich 1 oder 2 ist und p eine ganze Zahl gleich 2, 3 oder 5 bis 15 ist, wobei dieser Kohlenwasserstoff multizyklisch sein kann und eine Brücke aufweisen kann.

**13.** Verfahren nach Anspruch 12, dadurch gekennzeichnet, daß der zyklische ungesättigte Kohlenwasserstoff ein zyklisches Olefin, Dien, Trien oder Tetraen ist.

**14.** Verfahren nach Anspruch 13, dadurch gekennzeichnet, daß der zyklische ungesättigte Kohlenwasserstoff Cyclopenten, Cycloocten, Cyclodecen, Cyclododecen, Cyclooctadien, Cyclododecadien, Cyclododecatrien oder Cyclooctatetraen ist.

**15.** Verfahren nach Anspruch 12, dadurch gekennzeichnet, daß der zyklische ungesättigte Kohlenwasserstoff ein polyzyklisches Olefin oder Dien ist.

**16.** Verfahren nach Anspruch 15, dadurch gekennzeichnet, daß der polyzyklische ungesättigte Kohlenwasserstoff Norbornen, Norbornadien oder Dicyclopentandien ist.

**17.** Verfahren nach Anspruch 1, dadurch gekennzeichnet, daß der Katalysator der Formel (15) oder (16) an ein Halogenid oder eine metallorganische Verbindung von Ga, Al, Sn, Pb, Mg, Li, Ti oder B gebunden ist.

**18.** Verfahren nach Anspruch 1, dadurch gekennzeichnet, daß ein Katalysator der Formel (17) verwendet wird, der ein an eine metallorganische Pb- oder Sn-Verbindung gebundener $WCl_4(OAr)_2$-Komplex ist.

## Claims

**1.** A method for the preparation of an organic compound or polymer which contain each (i) one or several sulphur atoms in the sulphide form, and (ii) one or several double bonds in a hydrocarbon chain, by metathesis of an olefinic sulphide, which is cyclic or acyclic, characterised in that metathesis is carried out in the presence of a catalyst consisting of a tungsten complex containing at least one phenoxy group and selected from the group consisting of

(a) compounds of the formula :

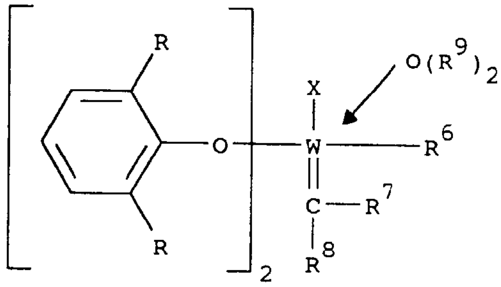

(15)

wherein X is halogen, R represents a hydrocarbon group or an electronegative atom, $R^6$ is alkyl, aryl or halogen, $R^7$ and $R^8$ are H atoms or alkyl groups and $R^9$ is an alkyl group ;

(b) compounds of the formula :

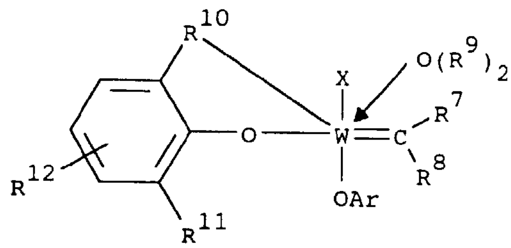

(16)

wherein $R^{11}$ and $R^{12}$ are each H or aryl, $R^{10}$ is a divalent arylene group, and Ar in the OAr group linked to W is aryl, X and $R^7$ to $R^9$ being defined as indicated above ; and,

(c) compounds of the formula :

$$WCl_{(6-x)}(OAr)_x \quad (17)$$

wherein OAr represents an aryloxy group, x is 2, 3 or 4, each compound of the formula 17 being associated with an organometallic compound of Ga, Al, Sn, Pb, Mg, Li, Ti or B.

**2.** A method according to claim 1 characterised in that the olefinic sulphide is subjected to a cross metathesis reaction with an acyclic olefin or an unsaturated cyclic non-aromatic sulphur-free hydrocarbon.

**3.** A method according to claim 1 characterised in that the olefinic sulphide is subjected to a cross metathesis reaction with itself.

**4.** A method according to claim 2 or 3 characterised in that the olefinic sulphide is an acyclic compound of the formula :

$$R^1\text{-}CH = CH\text{-}(CH_2)_n\text{-}S\text{-}R^2$$

wherein $R^1$ and $R^2$, which are identical or different, represent each H, $C_1$-$C_{12}$ alkyl, cycloalkyl, $C_2$-$C_{12}$ alkenyl or $C_6$-$C_{18}$ aryl, n being an integer of from 0 to 10.

**5.** A method according to claim 4 characterised in that the acyclic olefinic sulphide is allyl methyl sulphide.

**6.** A method according to claim 4 characterised in that the acyclic olefinic sulphide is allyl sulphide.

**7.** A method according to claim 2 or 3 characterised in that the olefinic sulphide is a cyclic compound which contains from 1 to 4 double bonds in its hydrocarbon chain and contains a thioalkyl group.

**8.** A method according to claim 7 characterised in that the cyclic olefinic sulphide is a compound of the formula :

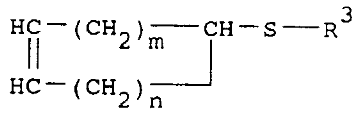

wherein m and n, which are identical or different, are integers of from 0 to 15, the sum m+n being different from 0 and different from 3, and $R^3$ is $C_1$-$C_{12}$ alkyl, $C_2$-$C_{12}$ alkenyl or $C_6$-$C_{18}$ aryl.

**9.** A method according to claim 8 characterised in that m is 2, n is 3 and $R^3$ is ethyl, n-butyl, tertiobutyl, hexyl or cyclohexyl.

**10.** A method according to claim 2 characterised in that the acyclic sulphur-free olefin, which used in the cross metathesis reaction. is of the formula : $R^4$-CH=CH-$R^5$, wherein $R^4$ and $R^5$, identical or different, are each H, $C_1$-$C_{10}$ alkyl, $C_2$-$C_{10}$ alkenyl or $C_6$-$C_{10}$ aryl.

**11.** A method according to claim 10 characterised in that $R^4$ and $R^5$ are simultaneously or independently H, $CH_3$ or $CH_2$-$CH_3$.

**12.** A method according to claim 2 characterised in that the unsaturated sulphur-free cyclic hydrocarbon used in the cross metathesis reaction, contains from 1 to 4 double bonds and is of the formula:

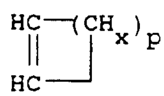

wherein x is 1 or 2 an p is an integer of from 2 to 3 or from 5 to 15, said hydrocarbon being able to be polycyclic and to include a bridge.

**13.** A method according to claim 12 characterised in that the unsaturated cyclic hydrocarbon is an olefin, a diene, a triene or a tetraene.

**14.** A method according to claim 13 characterised in that the unsaturated cyclic hydrocarbon is cyclopentene, cyclooctene, cyclodecene, cyclododecene, cyclooctadiene, cyclododecadiene, cyclododecatriene, or cyclooctatetraene.

**15.** A method according to claim 12 characterised in that the unsaturated cyclic hydrocarbon is a polycyclic olefin or diene.

**16.** A method according to claim 15 characterised in that the unsaturated polycyclic hydrocarbon is norbornene, norbornadiene or dicyclopentandiene.

**17.** A method according to claim 1 characterised in that the catalyst of the formula (15) or (16) is associated with a halide or an organometallic compound of Ga, Al, Sn, Pb, Mg, Li, Ti or B.

**18.** A method according to claim 1 characterised in that the catalyst of the formula (17), which is a $WCl_4(OAr)_2$ complex associated with an organometallic compound of Pb or Sn.